# EUROPÄISCHE PATENTSCHRIFT

(11) **EP 1 118 313 B1**
(45) Veröffentlichungstag und Bekanntmachung des Hinweises auf die Patenterteilung: **23.02.2005**
(21) Anmeldenummer: 00811206.2
(22) Anmeldetag: 19.12.2000
(51) Int. Cl.: A61F 2/46, B05C 17/015

(54) **Pistole zum Auspressen von Knochenzement mit einer aufsetzbaren Zementspritze**
Gun for squeezing out bone cement with a mountable cement syringe
Pistolet pour pressuriser du ciment à os avec une seringue à ciment amovible

(30) Priorität: 18.01.2000 EP 00810038
(43) Veröffentlichungstag der Anmeldung: 25.07.2001
(73) Patentinhaber: Zimmer GmbH, 8404 Winterthur (CH)
(72) Erfinder: Overes, Tom, 8400 Winterthur (CH); Faoro, Francisco, 8002 Zürich (CH)
(74) Vertreter: Manitz, Finsterwald & Partner GbR

(56) Entgegenhaltungen:
- EP-A- 0 108 584
- EP-A- 0 170 120
- DE-A- 4 022 986
- US-A- 2 818 999
- US-A- 3 768 472
- US-A- 5 431 654
- US-A- 5 514 135

## Beschreibung

Die Erfindung handelt von einer Pistole zum Auspressen von Knochenzement mit einer aufsetzbaren Zementspritze, welche einen Zylinder mit Ausstosskolben und einen an den Zylinder anschliessenden, schlankeren Hals aufweist, wobei in die Pistole ein Verdrängerstab einsetzbar ist, welcher nach dem Ausstossen des Zylinderinhalts mittels einer auf den Ausstosskolben wirkenden ersten rohrförmigen Stange mit einem in der rohrförmigen Stange gelagerten zweiten Vorschubmechanismus durch einen Durchbruch des Ausstosskolbens hindurch in den Hals einführbar ist.

Zementpistolen zum Auspressen von Knochenzement aus einer Zementspritze sind seit einigen Jahren im Gebrauch. In der Regel wird die Zementspritze auf der Pistole aufgesetzt und anschliessend über einen Abzugshebel eine Stange gegen den Ausstosskolben der Zementspritze vorgeschoben, um den Zement auszustossen. Eine solche Sperrklinken-Ausführung ist in einem Prospekt der Firma DePuy, International Ltd., St. Antony's Road, Beeston, Leeds, GB LS11 8DT, unter dem Titel CMW MKIII ZEMENTPISTOLE (1363-024) gezeigt. Sie hat die Besonderheit, dass die über den Abzugshebel und Sperrklinke betätigte Stange aus einem mit einer Zahnung versehenen Rohr besteht, an dessen Ende ein Fenster angebracht ist. Wenn dieses Fenster den Abzugsmechanismus erreicht, greift dieser durch das Fenster in die Zahnung einer weiteren in dem Rohr gelagerten Stange und treibt diese relativ zum Rohr weiter vorwärts. Die zweite Stange kann somit bei Zementspritzen, die einen an den Zylinder anschliessenden Hals aufweisen, mit einem im Rohr eingesteckten Verdrängerstab das Volumen im Hals ausstossen, wenn der mit dem Rohr betätigte Kolben am Ende seines Weges angekommen ist. Die Vorrichtung hat den Nachteil, dass sie sperrig ist, weil die beiden Stangen beim Beginn des Ausstosses um ihre volle Länge nach hinten vorstehen. Ein weiterer Nachteil ist, dass der Operateur von Hand die Auspressarbeit leisten muss. Selbst wenn die Bewegung der Stangen durch den Abzughebel untersetzt ist, muss er diesen doch viele Male betätigen.

In der Patentschrift U.S. 5,514,135 ist eine Einweg-Knochenzementspritze mit integriertem Pistolengriff und einer im Griff angebrachten CO₂-Patrone gezeigt. Mit dem Abzughebel wird eine kleine Druckkammer von der CO₂-Seite zur Beaufschlagungsseite eines freien Kolbens verschoben, um portionenweise komprimiertes CO₂ dem Kolben zuzuführen. Der Knochenzement wird durch eine verschliessbare, in der Mantelfläche der Zementspritze angebrachte Öffnung eingefüllt und kann nach dem Verschliessen dieser Öffnung über eine weitere Austrittsöffnung ausgestossen werden. Ein Nachteil dieser Anordnung besteht darin, dass sie bezüglich Werkstoffwahl sterilisierbar sein muss, aber nur einmal verwendbar ist. Ein weiterer Nachteil besteht darin, dass hier das Risiko für einen nicht erkennbaren Gasdurchbruch zum Knochenzement besteht, wenn der Kolben klemmt und/oder sein Dichtungsring bezüglich Gasdichtheit versagt. Ein Gasdurchbruch ist auch in kleinen Mengen nicht tragbar.

Aufgabe der Erfindung ist es eine leicht zu handhabende und für unterschiedliche Ausstossbedingungen geeignete Zementpistole zu schaffen. Diese Aufgabe wird gemäss Patentanspruch 1 dadurch gelöst, dass die rohrförmige Stange als Kolbenstange eines ersten in der Pistole von einem Fluid beaufschlagten Kolbens ausgeführt ist und dass der zweite Vorschubmechanismus einen zweiten von dem Fluid beaufschlagten Kolben aufweist, der den Verdrängerstab ausstösst, wobei beide Kolben über eine Steuereinrichtung in der Pistole mit dem unter Druck stehenden Fluid ansteuerbar sind.

Vorteilhafte Weiterbildungen der Erfindung ergeben sich durch die abhängigen Patentansprüche 2 bis 14.

Um die Machbarkeit einer vielseitig verwendbaren Pistole zum Auspressen von Knochenzement abzuklären, insbesondere für kompressible, gasförmige Fluide, waren Versuche notwendig.

Für eine Zementspritze mit einem langen aufgesetzten Hals, wie sie bei dem "retrograd" - Auffüllen einer Oberschenkelmarkraumhöhle verwendet wird, ergeben sich beispielsweise folgende Daten:

| Wartezeit nach dem Anrühren des Knochenzements [min] | 6:00 | 6:20 | 5:38 | 4:00 |
|---|---|---|---|---|
| Temperatur [°C] | 24,4 | 20,0 | 19,5 | 20,2 |
| Mittlere Ausstosskraft [N] | 750 | 700 | 650 | 500 |
| Ausfliessgeschwindigkeit [mm/s] | 2,2 | 5,6 | 7,5 | 10,0 |

Für eine Zementspritze mit einem kurzen Hals, wie sie bei dem "antegrad"-Auffüllen einer Knochenaushöhlung verwendet wird, ergaben sich beispielsweise folgende Daten:

| Wartezeit nach dem Anrühren des Knochenzements [min] | 3:15 | 5:30 |
|---|---|---|
| Temperatur [°C] | 20 | 20 |
| Ausstosskraft [N] | 130 | 240 |
| Ausfliessgeschwindigkeit [mm/s] | 10 | 10 |

Für das Auspressen des Knochenzements mit einem Verdrängerstab aus einem langen aufgesetzten Hals ergab sich beispielsweise eine Kraft von 25 Newton.

Die Steuereinrichtung und der erste sowie der zweite vom Fluid beaufschlagte Kolben sind so angeordnet und dimensioniert, dass die obigen Ausstossbedingungen erfüllbar sind. Das Fluid kann eine Flüssigkeit oder ein Gas sein. Diese können von extern über Schläuche an den Pistolengriff herangeführt und wieder abgeführt werden. Einfacher wird die Handhabung, wenn das Reservoir mit dem Fluid in die Pistole beispielsweise in Form von Gaspatronen im Griffteil integriert ist. CO₂-Patronen haben den Vorteil, dass das Fluid flüssig mit der Patrone einführbar ist und wenig Volumen beansprucht. Ausserdem fällt der Druck im Reservoir nicht zu schnell ab, solange noch flüssiges CO₂ vorhanden ist.

Die Erfindung hat den Vorteil, dass sich der Operateur voll auf die eigentliche Zementapplikation konzentrieren kann. Ausserdem ist das Gerät für unterschiedliche Zementspritzen, d.h. mit unterschiedlich langem Hals und mit oder ohne Verdrängerstab einsetzbar.

Weiterhin ist es notwendig, dass die Ausstossbewegung des zweiten Kolbens erst eingeleitet wird, wenn der erste Kolben den Ausstosskolben in seine Endstellung gebracht hat. Bei einer zu frühen Auslösung des zweiten Kolbens würde dieser über den Verdrängerstab den Ausfluss im Hals der Zementspritze verschliessen. Eine Lösung sieht vor, den Verdrängerstab selbst als mechanische Rückführung zu benutzen. Wenn der Hub des ersten Kolbens grösser als der mögliche Verschiebeweg des Ausstosskolbens ist und ein stirnseitiger Spalt zwischen Kolbenstange und Ausstosskolben besteht, kann über die Breite dieses Spaltes die volle Ausstosskraft zwischen Verdrängerstab und Ausstosskolben erzeugt werden. Diese Ausstosskraft genügt, um eine in der Durchtrittsöffnung für den Verdrängerstab angeordnete Membran oder Klappe mit dem Verdrängerstab auf die Seite zu schieben und diesen mit dem zweiten Kolben weiter in den Hals hinein zu schieben. Wenn kein Verdrängerstab eingesetzt ist, erhält der zweite Kolben zwar zwangsläufig in der Endstellung des ersten Kolbens eine Beaufschlagung durch das Fluid, aber er bewegt sich nur langsam entsprechend einer davorliegenden Drosselstelle bis zu einem Anschlag.

In einer anderen Lösung wird der zweite Kolben von Anfang an mit dem unter Druck stehenden Fluid beaufschlagt und der Verdrängerstab, der seinerseits durch die Membran am Ausstosskolben abgestützt ist, verhindert, dass der zweite Kolben sich relativ zum ersten Kolben bewegt. Dies bedingt, dass der Widerstand der Membran gegen Durchstossen deutlich grösser ist als die grösste Auspresskraft währen dem Auspressen des Knochenzements aus dem zylindrischen Teil der Zementspritze und dass die maximal erzeugbare Kraft beim Auftreffen des Ausstosskolbens auf dem Übergangsstück zum Hals der Zementspritze deutlich grösser ist als der Widerstand der Membran.

Eine weitere Möglichkeit eine Losbrechkraft am Verdrängerstab zu erzeugen besteht in einem Differenzialkolben zwischen dem ersten und dem zweiten Kolben, wobei der Differenzialkolben über eine kurze Strecke mit seiner zusätzlichen Kolbenfläche den Druck vom Fluid aufnimmt und diese zusätzliche Kraft direkt an den zweiten Kolben überträgt, der seinerseits den Verdrängerstab bewegt.

Die Steuerung des Fluidstroms geschieht vorteilhaft mit einem Abzugbügel, der mit einem Steuerschieber verbunden ist.

Bei einem Fluid, das gasförmig in die Steuereinrichtung gelangt, ist es schwierig einen konventionellen Steuerschieber zu verwenden, da wegen der kleinen Vorschubgeschwindigkeiten für den Ausstosskolben, die Drosselstellen für den Fluidstrom so klein gewählt werden müssen, dass der Einfluss vom Spiel des Steuerschiebers störend wirkt. Es hat sich daher als vorteilhaft erwiesen, einen Steuerschieber mit O-Ringen in einem Rohrstück zu lagern und in diesem Rohrstück in Längsrichtung einen Sperrbereich und Einzelbohrungen soweit voneinander anzubringen, dass sie nacheinander von einem O-Ring im Steuerschieber für den Durchfluss freigegeben werden. Da bei einem unter Druck an den Kolben anstehenden Gaspolster, dieses auch noch ansteht und Zement auspresst, wenn der Durchfluss von der Zuleitung gesperrt wird, ist es vorteilhaft, wenn bei nicht-betätigtem Abzugbügel, d.h. bei gesperrtem Zulauf gleichzeitig das noch anstehende Gaspolster zwangsläufig entlüftet wird.

Um dem Operateur taktil ein Gefühl für die Auspressgeschwindigkeit zu vermitteln, muss er zunächst den Abzugbügel aus dem Sperrbereich gegen eine weiche Feder in einen Langsamgangbereich bringen, bei dem eine kleine Einzelbohrung als Durchflussquerschnitt frei wird. Bei CO₂ kann dieser Bohrungsdurchmesser 0,08 mm betragen, um den Knochenzement nach dem Aufsetzen der Zementspritze langsam und vorbereitend bis zur Mündung der Spritze zu bewegen. Beim weiteren Durchziehen des Abzugbügels kommt eine steifere Feder in Eingriff, gleichzeitig wird die Anzahl der wirksamen Einzelbohrungen erhöht. Auf diese Weise ist es möglich, die Funktionen Sperren, Langsamgang und Auspressen bis zur Maximalgeschwindigkeit auf einem Verschiebeweg des Abzugbügels zu verteilen, der zwischen 10 und 20 mm betragen kann und als angenehm für die Einstellung empfunden wird.

Im folgenden wird die Erfindung anhand von Ausführungsbeispielen beschrieben. Es zeigen:
- Fig. 1: Schematisch einen Längsschnitt durch eine Zementpistole mit aufgesetzter Zementspritze;
- Fig. 2: schematisch einen Längsschnitt durch eine weitere Anordnung mit einem zusätzlichen Differenzialkolben;
- Fig. 3: schematisch eine Frontalansicht von Figur 2;
- Fig. 4: schematisch einen vergrösserten Ausschnitt der Steuereinrichtung von Figur 2, bei welchem der Abzughebel auf Langsamgang steht.
- Fig. 5: schematisch einen vergrösserten Ausschnitt analog zu Figur 1 mit einem zweiten Kolben, der gleichzeitig mit dem ersten Kolben vom Fluid beaufschlagt wird;
- Fig. 6: schematisch einen Ausschnitt aus Figur 1 mit einer Klappe im Durchbruch des Ausstosskolbens, welche durch den Verdrängerstab geöffnet ist.

Die Figuren zeigen eine Pistole zum Auspressen von Knochenzement 1 mit einer aufsetzbaren Zementspritze 2, welche einen Zylinder 3 mit Ausstosskolben 4 und einen an den Zylinder 3 anschliessenden, schlankeren Hals 5 aufweist, wobei in die Pistole ein Verdrängerstab 6 einsetzbar ist, um Restzement zusätzlich mit einem zweiten Vorschubmechanismus 8 auszustossen. Ein erster Kolben 9 wirkt über eine Kolbenstange 7 auf den Ausstosskolben 4. Ein zweiter Kolben 11, welcher in der Kolbenstange 7 verschiebbar gelagert ist, wirkt auf den Verdrängerstab 6. Ein unter Druck stehendes Fluid 10 wird über eine Steuereinrichtung 12 so gesteuert, dass sich der Verdrängerstab 6 relativ zum ersten Kolben 9 erst bewegt, wenn dieser eine vorgesehene Endstellung erreicht hat.

In den Figuren sind gleiche Hinweiszeichen für gleiche Funktionselemente verwendet. So sind O-Ringe generell mit dem Hinweiszeichen 30 versehen.

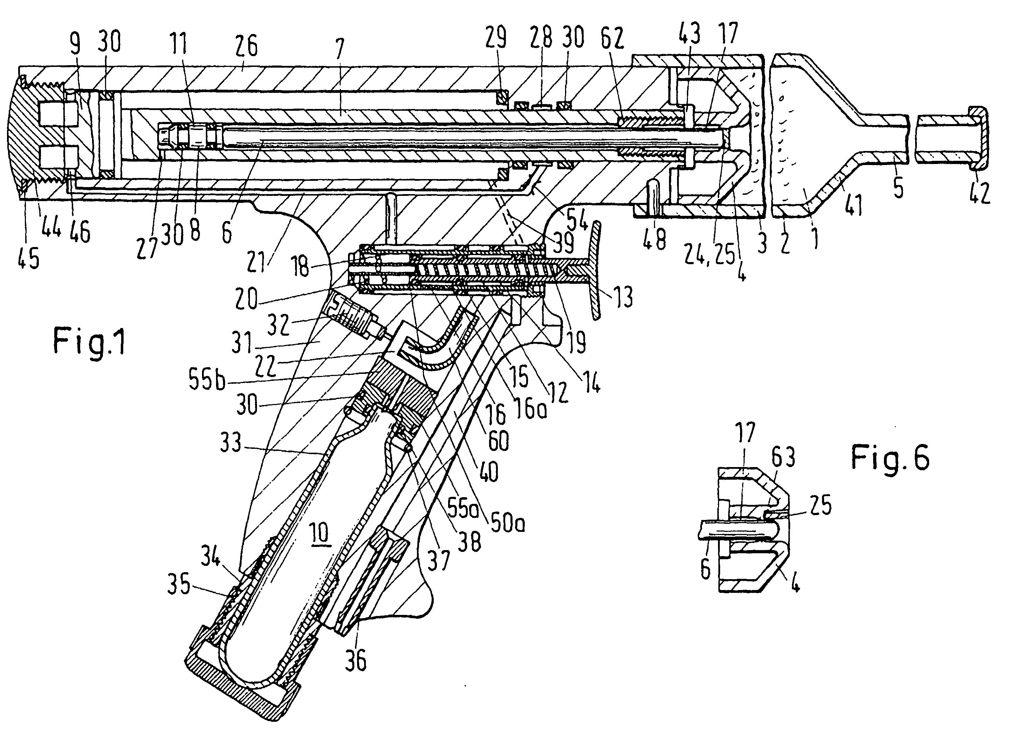

Im Beispiel von Figur 1 ist eine mit flüssigem Knochenzement 1 gefüllte Zementspritze 2 an ihrem Hals 5 mit einer Kappe 42 verschlossen. An ihrer Gegenseite ist die Zementspritze 2 an einem Zylinder 3 mit einem Ausstosskolben 4 verschlossen, der seinerseits einen Durchbruch 17 besitzt, welcher mit einer Membran 24 oder Klappe 25 verschlossen ist. Der Zylinder 3 ist mit einem Bajonettverschluss über Stifte 48 am Gehäuse 26 der Zementpistole befestigt. Im Gehäuse 26 ist ein erster Kolben 9 in Ausstossrichtung verschiebbar. Sein Hub wird in einer hinteren Endstellung durch einen Deckel 44 und in einer vorderen Endstellung durch einen Puffer 29 begrenzt. Die Ausstossbewegung des ersten Kolbens 9 wird auf eine Kolbenstange 7 übertragen, die gleichzeitig eine Längsbohrung aufweist, in der ein zweiter Kolben 11 in seiner hinteren Endstellung durch einen eingesteckten Verdrängerstab 6 gehalten ist. Das andere Ende des Verdrängerstabes 6 ragt aus der Kolbenstange 7 in einen Durchbruch 17 des Ausstosskolbens 4 hinein und hat Kontakt mit einer Membran 24 oder einer Klappe 25, die den Durchbruch 17 abdeckt.

Zwischen der Stirnseite der Kolbenstange 7 und dem Ausstosskolben 4 besteht ein Spalt 43. Bei einer Ausstossbewegung der ersten Kolbens 9 stösst der Verdrängerstab 6 zunächst gegen die Membran 24 oder eine Klappe 25 im Durchbruch 17 des Ausstosskolbens 4, macht den Durchbruch 17 auf und verschliesst ihn gleichzeitig mit seinem Querschnitt. Falls der Widerstand von der Membran 24 oder der Klappe 25 zu gross ist, wird der Durchbruch 17 erst durchstossen, wenn der Ausstosskolben 4 an einem konischen Übergang 41 der Zementspritze 2 auftrifft. Der Hub des ersten Kolbens 9 ist so bemessen, dass er seine vordere Endstellung erst erreicht, wenn der Spalt 43 aufgehoben und der Durchbruch 17 durchstossen ist. In Figur 7 ist eine Klappe 25 mit einem Filmscharnier 63 im Durchbruch 17 des Ausstosskolbens 4 befestigt, welche mit einer relativ geringen Axialkraft vom Verdrängerstab 6 geöffnet werden kann. Ein Verschluss des Durchbruchs 17 in Form einer Membran 24 oder einer Klappe 25 ist nur dann zwingend, wenn mit der gleichen Anordnung auch ohne Verdrängerstab 6 gearbeitet wird. In einem solchen Fall würde als Verschluss auch ein Stopfen im Durchbruch 17 genügen.

Wenn der erste Kolben 9 seine vordere Endstellung erreicht hat und der Durchbruch 17 des Ausstosskolbens vom Verdrängerstab 6 durchstossen ist, hat eine radiale Bohrung 27 der Kolbenstange 7, welche hinter den zweiten Kolben 11 in seiner hinteren Endstellung führt, einen Ringraum 28 im Gehäuse 26 erreicht, welcher über O-Ringe 30 abgedichtet ist. Dieser Ringraum 28 wird ebenfalls über das unter Druck stehende Fluid 10 angespeist, wobei eine Drosselstelle 54 den Fluidstrom und somit die Vorschubgeschwindigkeit für den Verdrängerstab 6 beim Eintauchen in den Hals 5 der Zementspritze bestimmt. Mit oder ohne Verdrängerstab 6 bewegt sich der zweite Kolben 11 bis zu einem vorderen Anschlag 62, der als einschraubbare Hülse ausgeführt ist.

Nach dem Auspressen des Knochenzements können Zementspritze 2 und Verdrängerstab 6 von der Zementpistole entfernt werden und der zweite und der erste Kolben 11, 9 in ihre hintere Endstellung zurückgestossen werden. Je nach Kapazität der Druckquelle für das Fluid 10 kann ein neuer Verdrängerstab 6 und eine neue Knochenzementspritze 2 sofort auf die Pistole aufgesetzt werden.

An das Gehäuse 26 ist ein Griffteil 31 angeschlossen, in welchem eine Patrone 33 mit einem unter Druck stehenden Fluid 10, zum Beispiel CO₂, eine Steuereinrichtung 12 und ein Anschluss 36 für den Wegtransport des verbrauchten Fluids vorgesehen ist. Die Patrone 33 wird über einen Einsatz 34 und eine Haltemutter 35 in ihrer Position gehalten. Zwei Ringstücke 55a, 55b sind mit Sprengring 37 im Griffteil gehalten. Das Ringstück 55a dichtet mit O-Ringen zwischen dem Hals der Patrone 33 und dem Griffteil 31. Das Ringstück 55b sticht mit einer hohlen Schneide 38 eine Verschlussfolie der Patrone 33 an und setzt das Fluid frei. Das Fluid gelangt zunächst in eine Zuleitung 22, die im Fall von CO₂ zu einer Vorkammer erweitert ist in welche ein gebogenes Röhrchen 60 hineinragt. Dies hat den Vorteil, dass auch bei vertikal nach unten stehender Pistole das flüssig aus der Patrone 33 kommende CO₂ in der Vorkammer verdampft und als Gas der Steuereinrichtung 12 zugeführt wird, ohne dass Flüssigkeitsreste in die Steuereinrichtung 12 gelangen. Die Vorkammer kann vor dem Entfernen der Patrone 33 mit einer Entlüftungsschraube 32 entlüftet werden.

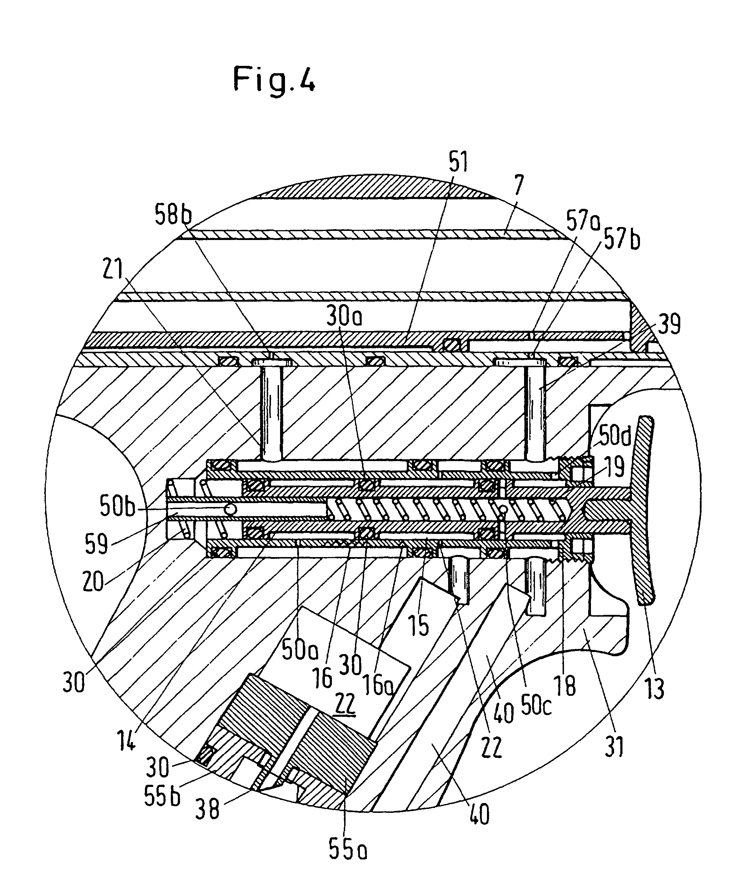

Die eigentliche Steuereinrichtung wird später mit Figur 4 beschrieben. Das Fluid gelangt nach der Steuereinrichtung 12 in eine Verbindungsleitung 21 und von dort über eine Bohrung 46 zum ersten Kolben 9 sowie über die Drosselstelle 54 und den Ringraum 28 an die Kolbenstange 7. Der Raum vor dem ersten Kolben 9 zur Kolbenstange 7 hin ist über eine Rücklaufleitung 39 durch die Steuereinrichtung 12 hindurch zu einer Rücklaufleitung 40 entlüftet, sodass sich kein Druck aufbauen kann, der durch Bohrung 27 hindurch den zweiten Kolben 11 bewegt. Erst wenn die Bohrung 27 den Ringraum 28 erreicht hat, gelangt das Fluid zum zweiten Kolben 11.

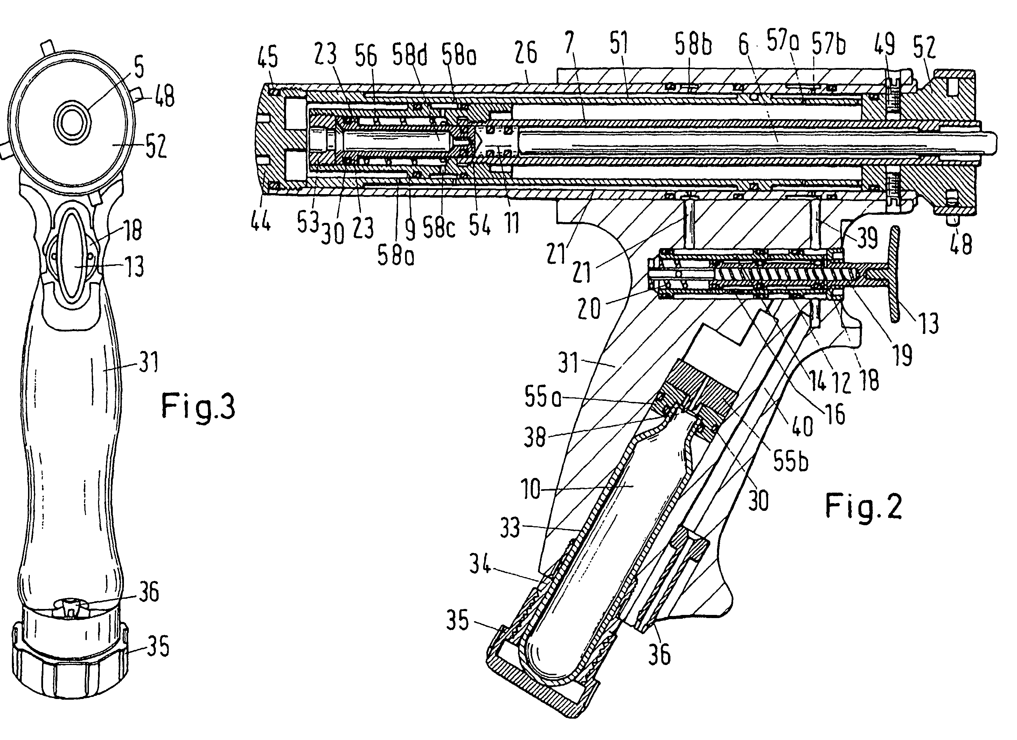

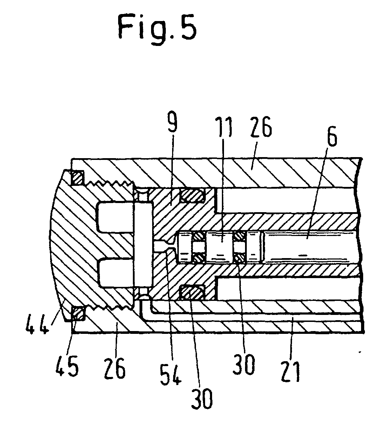

Figur 5 zeigt eine Variante zu Figur 1, bei der auf einen Ringraum 28 verzichtet wird und bei der der zweite Kolben 11 direkt durch den ersten Kolben 9 hindurch über eine Drosselstelle 54 angespeist wird. Dies bedingt, dass der Verdrängerstab 6 während dem Ausstossen des Knochenzements aus dem Zylinder 3 der Zementspritze den Ausstosskolben 4 nicht durchbricht. Aus diesem Grund müsste eine Membran 24 relativ stark dimensioniert sein. Eine weitere Lösung mit einer schwach zu dimensionierenden Membran 24 ist in Figur 2 gezeigt.

In Figur 2 und 3 sind Gehäuse 26 und Griffteil 31 getrennt und über Bolzen 49 verbunden, welche gleichzeitig einen Kopfteil 52 fixieren, der die Stifte 48 für den eigentlichen Bajonettverschluss einer Zementspritze 2 trägt. Zwischen einem Deckel 44, der mit einer Dichtung 45 gegen das Gehäuse 26 abgedichtet ist, und dem Kopfteil 52 ist ein separater Zylinder 51 gefangen, in welchem ein erster Kolben 9 läuft, dessen Kolbenstange 7 aus dem Kopfteil 52 vorsteht, um direkt auf einen Ausstosskolben 4 zu wirken. In der hohlen Kolbenstange 7 ist ein zweiter Kolben 11 gelagert, der auf einen einsteckbaren Verdrängerstab 6 wirken kann. Dieser zweite Kolben 11 steht von Anfang an mit dem ersten Kolben 9 unter dem Druck des Fluids, welches von der Steuereinrichtung 12 über Verbindungsleitungen 21 und Bohrungen 58a, 58b dem ersten Kolben 9 und weiter über eine Drosselstelle 54 dem zweiten Kolben 11 zugeführt wird. Der Querschnitt des zweiten Kolbens 11 ist relativ gering, sodass eine Membran 24 im Ausstosskolben 4, welche den zweiten Kolben 11 über den Verdrängerstab 6 blockiert relativ schwach bemessen sein darf, da sie auf der Gegenseite von dem unter Auspressdruck stehenden Knochenzement gestützt ist.

Innerhalb des ersten Kolbens 11 ist ein Differenzialkolben 23 angeordnet, der mit seinem kleineren Kolben dem Durchmesser des zweiten Kolbens 11 entspricht, der in die hohle Kolbenstange 7 hineinragt und der die Drosselstelle 54 zum zweiten Kolben 11 enthält. Der Differenzialkolben 23 wird durch eine Feder 56 in seine hintere Endstellung gegen eine durchbrochene Verschlussschraube 53 gepresst. Da der Differenzialkolben 23 von allen Seiten mit dem gleich hohen Druck des Fluids umgeben ist, behält er diese Endlage im ersten Kolben 9 bei, solange sich die Druckverhältnisse nicht ändern. Erst wenn der erste Kolben 9, welcher den Ausstosskolben 4 direkt über die Kolbenstange 7 ausstösst, seine vordere Endstellung erreicht hat, ändern sich die Druckverhältnisse, da eine Bohrung 58c über Bohrungen 57a und 57b mit einer drucklosen Rücklaufleitung 39 kurz geschlossen wird. Bohrungen 58d im kleineren Durchmesser des Differenzialkolbens 23 sind so klein bemessen, dass der Druck im Ringraum vor dem Differenzialkolben durch das Entlüften abfällt. Der Differenzialkolben 23 wird daher entsprechend dem Druckunterschied auf der Ringfläche in Ausstossrichtung gestossen und erzeugt am zweiten Kolben 11 eine zusätzliche Kraft, die ausreicht um die Membran 24 mit dem Verdrängerstab 6 zu durchstossen. Für das anschliessende Eintauchen des Verdrängerstabes 6 in einen Hals 5 einer Zementspritze 2 ist die Kolbenfläche des zweiten Kolbens 11 ausreichend.

Eine Patrone 33 als Reservoir für ein Fluid 10 ist analog zu Figur 1 im Griffteil 31 eingebaut; ebenso eine Rücklaufleitung 40 mit einem Anschluss 36. Die Steuereinrichtung 12 ist gleich wie im Beispiel von Figur 1 und wird nachfolgend beschrieben.

Die vergrösserte Darstellung in Figur 4 zeigt die Steuereinrichtung 12. Im Griffteil 31 ist ein Rohrstück 18 eingebaut, welches auf seiner Innenseite einen Steuerschieber 14 führt, der mit einem Abzugbügel 13 versehen ist. Das Rohrstück 18 ist auf seiner Aussenseite durch O-Ringe 30 in unterschiedliche Zonen unterteilt. Eine erste Zone ist mit der Rücklaufleitung 40 verbunden. Eine weitere, zweite Zone ist mit der Zuleitung 22 verbunden und eine dritte Zone ist mit der Verbindungsleitung 21 für die Kolben 9, 11 verbunden. Auf der Innenseite des Rohrstücks 18 bildet der Steuerschieber 14 mit O-Ringen 30 zwei verschiebbare Kammern zum Rohrstück 18, welche durch einen O-Ring 30a getrennt sind, der eine Art Steuerkante bildet. Längs des Rohrstücks 18 besteht für diesen O-Ring 30a ein Sperrbereich 15 ohne Bohrung in der Mantelfläche, daran angrenzend eine Einzelbohrung 16a für einen Langsamgang und in einem Abstand daran anschliessend mehrere Einzelbohrungen 16. Bei unbetätigtem Steuerschieber 14 ist dieser durch eine erste, weiche Rückstellfeder 19 in seiner äussersten rechten Position gehalten, in welcher der O-Ring 30a die Zuleitung 22 von der Verbindungsleitung 21 trennt. Bei einer Betätigung des Abzugbügels 13 wird zunächst die Einzelbohrung 16a für den Langsamgang freigegeben, wobei der Querschnitt der Einzelbohrung die Auspressgeschwindigkeit des Ausstosskolbens 4 oder des Verdrängerstabes 6 mitbestimmt. Für eine C02-Patrone beträgt der Durchmesser der Einzelbohrung 16a 0,08 mm.

Wenn der Abzugbügel 13 weiter bewegt wird, entsteht die in Figur 4 gezeichnete Lage bei der weitere Einzelbohrungen 16 freigegeben werden, wobei durch den Zuwachs an Durchtrittsflächen eine grössere Ausstossgeschwindigkeit entsteht. Der Vorteil dieser Einrichtung besteht darin, dass durch die O-Ringe 30, 30a praktisch keine Spielverluste auftreten und dass der Zuwachs an Durchtrittsfläche, trotz der kleinen Querschnitte, auf eine längere Strecke verteilt werden kann, die dem Bewegungsbereich eines Abzugfingers optimal anpassbar ist. Eine weitere taktile Hilfe ist eine zweite Rückstellfeder 20, die beim Übergang von der Einzelbohrung 16a zu den weiteren Einzelbohrungen 16 zusätzlich in Eingriff kommt und so ein Gefühl für die Grösse des Durchtrittsquerschnitts resp. für die Ausstossgeschwindigkeit vermittelt. In Figur 2 ist der Steuerschieber 14 in seiner äussersten rechten Position gezeichnet. In dieser Lage "unbetätigt" kommen Bohrungen 50c und 50d zur Deckung, sodass der Innenraum, in welchem die erste Rückstellfeder 19 gelagert ist, zur Rücklaufleitung 40 entlüftet wird. Gleichzeitig ist in dieser Stellung die Verbindungsleitung 21 über Bohrungen 50a und 50b mit diesem Innenraum verbunden und wird ebenfalls entlüftet. Dies hat für die Bedienung zur Folge, dass bei jedem Unterbrechen des Auspressvorgangs das Druckpolster zwischen Steuerschieber 14 und Kolben 9, 11 abgebaut wird, um ein unbeabsichtigtes Nachpressen zu verhindern.

## Patentansprüche

1. Pistole zum Auspressen von Knochenzement (1) mit einer aufsetzbaren Zementspritze (2), welche einen Zylinder (3) mit Ausstosskolben (4) und einen an den Zylinder anschliessenden, schlankeren Hals (5) aufweist, wobei in die Pistole ein Verdrängerstab (6) einsetzbar ist, welcher nach dem Ausstossen des Zylinderinhalts mittels einer auf den Ausstosskolben (4) wirkenden ersten rohrförmigen Stange (7) mit einem in der rohrförmigen Stange (7) gelagerten zweiten Vorschubmechanismus (8) durch einen Durchbruch (17) des Ausstosskolbens hindurch in den Hals (5) einführbar ist, **dadurch gekennzeichnet, dass** die rohrförmige Stange (7) als Kolbenstange (7) eines ersten in der Pistole von einem Fluid (10) beaufschlagten Kolbens (9) ausgeführt ist und dass der zweite Vorschubmechanismus einen zweiten von dem Fluid (10) beaufschlagten Kolben (11) aufweist, der den Verdrängerstab (6) ausstösst, wobei beide Kolben (9, 11) über eine Steuereinrichtung (12) in der Pistole mit dem unter Druck stehenden Fluid (10) ansteuerbar sind.

2. Pistole nach Anspruch 1, **dadurch gekennzeichnet, dass** das Fluid (10) ein Gas ist.

3. Pistole nach Anspruch 2, **dadurch gekennzeichnet, dass** das Gas aus einem Reservoir in der Pistole, beispielsweise aus einer auswechselbaren CO₂- oder NO₂-Patrone stammt.

4. Pistole nach einem der Ansprüche 1 bis 3, **dadurch gekennzeichnet, dass** der zweite Kolben (11) solange blockiert ist, bis der erste Kolben (9) eine vorgesehene vordere Endstellung erreicht hat.

5. Pistole nach Anspruch 4, **dadurch gekennzeichnet, dass** der Verdrängerstab (6) derart im Ausstosskolben (4) blockiert ist, dass er erst bei einer vorgesehenen Losbrechkraft den Ausstosskolben (4) durchstösst.

6. Pistole nach Anspruch 4, **dadurch gekennzeichnet, dass** der zweite Kolben (11) erst mit Druck beaufschlagbar ist, wenn der erste Kolben (9) seine vorgesehene vordere Endstellung erreicht hat.

7. Pistole nach einem der Ansprüche 1 bis 6, **dadurch gekennzeichnet, dass** die Steuereinrichtung (12) einen Abzugbügel (13) aufweist, der auf einen Steuerschieber (14) zur Dosierung des unter Druck stehenden Fluids (10) einwirkt.

8. Pistole nach Anspruch 7, **dadurch gekennzeichnet, dass** der Steuerschieber ein Rohrstück (18) mit einem Sperrbereich (15) und mit einem Bereich mit Einzelbohrungen (16) abdeckt, welche nacheinander über den Abzugbügel (13) in den Fluidstrom einbringbar sind, um stufenweise die Ausstossgeschwindigkeit am ersten Kolben (9) zu erhöhen.

9. Pistole nach Anspruch 8, **dadurch gekennzeichnet, dass** mit der Vergrösserung des Durchflussquerschnitts für den Fluidstrom eine wachsende Rückstellkraft am Abzugbügel (13) spürbar ist.

10. Pistole nach Anspruch 9, **dadurch gekennzeichnet, dass** eine erste weiche Rückstellfeder (19) im Bereich eines vorgesehenen kleinsten Durchflussquerschnitts (16a) wirksam ist und dass eine härtere zweite Rückstellfeder (20) in dem Bereich mit einem grösseren Durchflussquerschnitt (16) wirksam ist.

11. Pistole nach einem der Ansprüche 8 bis 10, **dadurch gekennzeichnet, dass** bei unbetätigtem Abzugsbügel (13) durch den Steuerschieber (14) die Verbindungsleitung (21) zwischen dem Steuerschieber (14) und dem ersten Kolben (9) entlüftet ist und die Zuleitung (22) vom Reservoir (10) zum Steuerschieber (14) gesperrt ist.

12. Pistole nach Anspruch 5, **dadurch gekennzeichnet, dass** innerhalb des ersten Kolbens (9) als dritter Kolben ein Differenzialkolben (23) gelagert ist, der auf den zweiten Kolben (11) wirkt, um über seine grössere Kolbenfläche eine grössere Losbrechkraft als mit dem zweiten Kolben (11) herstellbar zu erzeugen.

13. Pistole nach Anspruch 5, **dadurch gekennzeichnet, dass** der Verdrängerstab (6) durch eine Membran (24) im Durchbruch (17) des Ausstosskolbens (4) in axialer Richtung blockiert ist, wobei die Membran (24) bei einer vorgesehenen Losbrechkraft des Verdrängerstabes (6) perforierbar ist, um letzterem den Weg in Ausstossrichtung freizugeben.

14. Pistole nach Anspruch 5 oder 6, **dadurch gekennzeichnet, dass** im Durchbruch (17) des Ausstosskolens (4) eine Klappe (25) angebracht ist, die den Knochenzement (1) am Ausfliessen hindert und die dem Verdrängerstab (6) den Weg in Ausstossrichtung freigibt.

## Claims

1. Pistol for the pressing out of bone cement (1), including an attachable cement syringe (2) which has a cylinder (3) with an ejection piston (4) and a narrower neck (5) which adjoins the cylinder, with a displacer bar (6) being insertable into the pistol, which displacer bar (6), after the ejection of the cylinder content, can be introduced into the neck (5) through an aperture (17) of the ejection piston by means of a first tubular bar (7) which acts on the ejection piston (4) and which has a second advance mechanism (8) which is supported in the tubular bar (7), **characterized in that** the tubular bar (7) is designed as a piston rod (7) of a first piston (9) which is acted on by a fluid (10) in the pistol; and **in that** the second advance mechanism has a second piston (11) which is acted on by the fluid (10) and which ejects the displacer bar (6), with the two pistons (9, 11) being controllable by the fluid (10), which is under pressure, via a control device (12) in the pistol.

2. Pistol in accordance with claim 1, **characterized in that** the fluid (10) is a gas.

3. Pistol in accordance with claim 2, **characterized in that** the gas stems from a reservoir in the pistol, for example from a replaceable CO₂ or NO₂ cartridge.

4. Pistol in accordance with any one of the claims 1 to 3, **characterized in that** the second piston (11) is blocked until the first piston (9) has reached a predetermined front end position.

5. Pistol in accordance with claim 4, **characterized in that** the displacer bar (6) is blocked in the ejection piston (4) such that it punctures the ejection piston (4) only at a predetermined breaking-loose force.

6. Pistol in accordance with claim 4, **characterized in that** the second piston (11) can be acted on with pressure only when the first piston (9) has reached its predetermined front end position.

7. Pistol in accordance with any one of the claims 1 to 6, **characterized in that** the control device (12) has a trigger (13) which acts on a control slider (14) for metering the fluid (10) which is under pressure.

8. Pistol in accordance with claim 7, **characterized in that** the control slider covers a tube piece (18) with a blocking region (15) and with a region with individual bores (16) which can be brought one after the other into the fluid flow via the trigger (13) in order to step-wise increase the ejection speed at the first piston (9).

9. Pistol in accordance with claim 8, **characterized in that** an increasing restoring force can be felt at the trigger (13) with the increasing of the through-flow cross-section for the fluid flow.

10. Pistol in accordance with claim 9, **characterized in that** a first weak restoring spring (19) is effective in the region of a predetermined smallest through-flow cross-section (16a); and **in that** a stiffer, second restoring spring (20) is effective in the region with a greater through-flow cross-section (16).

11. Pistol in accordance with any one of the claims 8 to 10, **characterized in that**, when the trigger (13) is not actuated, the connection line (21) between the control slider (14) and the first piston (9) is ventilated by the control slider (14) and the inflow line (22) from the reservoir (10) to the control slider (14) is blocked.

12. Pistol in accordance with claim 5, **characterized in that** a differential piston (23), which acts on the second piston (11), is supported inside the first piston (9) as a third piston in order to produce a greater breaking-loose force via its greater piston surface than can be produced with the second piston (11).

13. Pistol in accordance with claim 5, **characterized in that** the displacer bar (6) is blocked in the axial direction by a membrane (24) in the aperture (17) of the ejection piston (4), with the membrane (24) being able to be perforated at a predetermined breaking-loose force of the displacer bar (6) in order to free the way for the latter in the ejection direction.

14. Pistol in accordance with claim 5 or claim 6, **characterized in that** a flap (25) is attached in the aperture (17) of the ejection piston (4) which hinders the bone cement (1) from flowing out and which frees the way for the displacer bar (6) in the ejection direction.

## Revendications

1. Pistolet pour l'éjection sous pression de ciment pour os (1), comprenant une seringue à ciment (2) amovible, présentant un cylindre (3) avec piston d'expulsion (4) et un col (5) plus mince qui fait suite au cylindre, dans lequel une barre de poussée (6) peut être insérée dans le pistolet et, après l'expulsion du contenu du cylindre, elle peut être guidée, à l'aide d'une première tige (7) de forme tubulaire agissant sur le piston d'expulsion (4), par un deuxième mécanisme d'avancement (8) monté dans la tige (7) de forme tubulaire, dans une ouverture (17) prévue dans le piston d'expulsion pour arriver jusque dans le col (5), **caractérisé en ce que** la tige (7) de forme tubulaire est réalisée en tant que tige de piston (7) d'un premier piston (9) sollicité dans le pistolet par un fluide (10), et **en ce que** le deuxième mécanisme d'avancement présente un deuxième piston (11) sollicité par le fluide (10) et qui expulse la barre de poussée (6), les deux pistons (9, 11) pouvant être commandés par le fluide sous pression (10), par l'intermédiaire d'un dispositif de commande (12), présent dans le pistolet.

2. Pistolet selon la revendication 1, **caractérisé en ce que** le fluide (10) est un gaz.

3. Pistolet selon la revendication 2, **caractérisé en ce que** le gaz provient d'un réservoir prévu dans le pistolet, par exemple une cartouche de CO₂ ou de NO₂, qui est remplaçable.

4. Pistolet selon l'une des revendications 1 à 3, **caractérisé en ce que** le deuxième piston (11) reste bloqué jusqu'à ce que le premier piston (9) ait atteint une position finale avant prévue.

5. Pistolet selon la revendication 4, **caractérisé en ce que** la barre de poussée (6) est bloquée de telle manière dans le piston d'expulsion (4) que ce soit lors d'une force prévue de rupture qu'elle passe à travers le piston d'expulsion (4).

6. Pistolet selon la revendication 4, **caractérisé en ce que** le deuxième piston (11) ne peut être sollicité par une pression que lorsque le premier piston (9) a atteint sa position finale avant prévue.

7. Pistolet selon l'une des revendications 1 à 6, **caractérisé en ce que** le dispositif de commande (12) présente une manette de commande de décharge (13), qui agit sur un tiroir de distribution (14) pour le dosage du fluide sous pression (10).

8. Pistolet selon la revendication 7, **caractérisé en ce que** le tiroir de distribution recouvre un élément tubulaire (18) par une zone de blocage (15) et par une zone comportant des alésages individuels (16), qui peuvent être introduits successivement, par l'intermédiaire de la manette de commande de décharge (13), dans le flux de fluide, afin d'augmenter par paliers la vitesse d'expulsion au niveau du premier piston (9).

9. Pistolet selon la revendication 8, **caractérisé en ce que**, lorsque la section transversale de passage pour le flux de fluide augmente, une force croissante de rappel peut être détectée, au niveau de la manette de commande de décharge (13).

10. Pistolet selon la revendication 9, **caractérisé en ce qu'**un premier ressort de rappel (19) souple agit dans la zone d'une section transversale de passage (16a) la plus petite prévue, et **en ce qu'**un second ressort de rappel (20) plus dur agit dans la zone ayant une plus grande section transversale de passage (16).

11. Pistolet selon l'une des revendications 8 à 10, **caractérisé en ce que**, lorsque la manette de commande de décharge (13) n'est pas actionnée, la conduite de liaison (21) entre le tiroir de distribution (14) et le premier piston (9) est purgée de l'air, et la conduite d'amenée (22), s'étendant du réservoir (10) jusqu'au tiroir de distribution (14), est fermée, par le tiroir de distribution (14).

12. Pistolet selon la revendication 5, **caractérisé en ce qu'**un piston différentiel (23) est monté, en tant que troisième piston, à l'intérieur du premier piston (9), et **en ce qu'**il agit sur le deuxième piston (11) afin de produire, par l'intermédiaire de sa plus grande surface de piston, une force de rupture plus élevée que celle qui peut être produite par le deuxième piston (11).

13. Pistolet selon la revendication 5, **caractérisé en ce que** la barre de poussée (6) est bloquée dans le sens axial par une membrane (24) située dans l'ouverture (17) du piston d'expulsion (4), la membrane (24) pouvant être perforée lors de l'application d'une force prévue de rupture par la barre de poussée (6), afin de permettre la course de celle-ci dans le sens de l'expulsion.

14. Pistolet selon la revendication 5 ou 6, **caractérisé en ce qu'**un clapet (25) est placé dans l'ouverture (17) du piston d'expulsion (4), pour empêcher le ciment pour os (1) de s'échapper et pour permettre la course de la barre de poussée (6) dans le sens de l'expulsion.
